# EUROPÄISCHE PATENTSCHRIFT

(11) **EP 2 902 742 B1**
(45) Veröffentlichungstag und Bekanntmachung des Hinweises auf die Patenterteilung: **11.03.2020**
(21) Anmeldenummer: 15450004.5
(22) Anmeldetag: 26.01.2015
(51) Int. Cl.: G01B 11/24, G01B 21/04, G01B 5/012, A61B 5/107, A61B 5/00

(54) **Verfahren zum Kalibrieren und Betreiben einer Vorrichtung zum Erfassen der dreidimensionalen Geometrie von Objekten**
Method for calibrating and operating a device for detecting the three-dimensional geometry of objects
Procédé d'étalonnage et de fonctionnement d'un dispositif destiné à enregistrer la géométrie tridimensionnelle d'objets

(30) Priorität: 29.01.2014 DE 102014101070
(43) Veröffentlichungstag der Anmeldung: 05.08.2015
(73) Patentinhaber: a.tron3d GmbH, 9020 Klagenfurt am Wörthersee (AT)
(72) Erfinder: Jesenko, Jürgen, 9584 Finkenstein (AT); Blassnig, Andreas, 9020 Klagenfurt (AT)
(74) Vertreter: Beer & Partner Patentanwälte KG

(56) Entgegenhaltungen:
- WO-A1-2013/116880
- GB-A- 2 499 660
- US-B2- 7 068 825

## Beschreibung

Die Erfindung betrifft ein Verfahren zum Kalibrieren einer Vorrichtung zum optischen Erfassen der dreidimensionalen Geometrie von Objekten, wobei optische Eigenschaften von optischen Elementen der Vorrichtung vor dem Erfassen gemessen und auf einem Speichermedium hinterlegt werden, wodurch ein erstes Kalibrierdatenset erzeugt wird, und wobei das erste Kalibrierdatenset während des Erfassens der dreidimensionalen Geometrie des Objektes verwendet wird, um durch die optischen Elemente aufgenommene und/oder projizierte zweidimensionale Oberflächencharakteristika des Objektes rechnerisch zu entzerren.

Die Erfindung betrifft weiters ein Verfahren zum Betreiben einer Vorrichtung zum optischen Erfassen der dreidimensionalen Geometrie von Objekten, wobei optische Eigenschaften von optischen Elementen der Vorrichtung vor dem Erfassen gemessen und auf einem Speichermedium hinterlegt wurden, wodurch ein erstes Kalibrierdatenset erzeugt wurde, und wobei das erste Kalibrierdatenset während des Erfassens der dreidimensionalen Geometrie des Objektes verwendet wird, um durch die optischen Elemente aufgenommene und/oder projizierte zweidimensionale Oberflächencharakteristika des Objektes rechnerisch zu entzerren.

Die Erfindung betrifft weiters eine Vorrichtung zum optischen Erfassen der dreidimensionalen Geometrie von Objekten, insbesondere intra-oral Scanner, mit einem Speichermedium, wobei das Speichermedium wenigstens zwei Kalibrierdatensets enthält.

Vorrichtungen mit Speichermedien, die zwei oder mehr Kalibrierdatensets enthalten sind beispielsweise aus den Veröffentlichungen mit den Nummern US 7,068,825 B2 und GB 2 499 660 A bekannt.

Das optische Erfassen einer Oberflächengeometrie hat insbesondere in der Zahnmedizin, aber beispielsweise auch dem Werkzeugbau, in den letzten Jahren andere Methoden der Oberflächenerfassung, wie beispielsweise den Zahnabdruck, abgelöst und wird ständig weiterentwickelt. Insbesondere im Dentalbereich versucht man dabei gleichzeitig mehrere Ziele zu verfolgen. Neben der Miniaturisierung der Messgeräte, um für Mediziner bzw. Dentaltechniker und Patienten größtmöglichen Komfort zu erzielen, und einer Verbesserung der verschiedenen Rechenabläufe, um schnellstmöglich die erfassten Oberflächengeometrien darzustellen und für weitere Verwendung zur Verfügung zu stellen, liegt ein weiteres Bestreben darin, die Geometrien immer genauer zu erfassen, also eine möglichst geringe Abweichung zwischen der realen Oberflächengeometrie des Objektes und der erfassten Oberflächengeometrie zu erreichen. Dabei sind sowohl eine möglichst korrekte Form als auch ein möglichst korrekter Maßstab entscheidend. Zu diesem Zweck ist es üblich, die Vorrichtungen zum Erfassen der dreidimensionalen Oberflächengeometrien von Objekten regelmäßig zu kalibrieren, um so Fehlern in den Messungen, welche durch die Optiken verursacht werden können, beispielsweise Linsenverzerrungen, auszugleichen und damit zu entzerren. Dafür sind allerdings häufig gesonderte Vorrichtungen notwendig, die zusätzliche Kosten verursachen. Darüber hinaus ist für ein solches regelmäßiges Kalibrieren Arbeitszeit erforderlich, welche die Betriebskosten zum Betreiben der Vorrichtungen erhöht. Dem gegenüber stehen Vorrichtungen, welche lediglich einmal, im Rahmen der Fertigung, kalibriert werden. Bei diesen kann, beispielsweise durch den Verzicht auf bewegliche Teile, wie zum Beispiel Einrichtungen zum Fokussieren, ein weiteres oder wiederholtes bzw. reguläres Kalibrieren vermieden werden. Allerdings kann mit solchen Vorrichtungen nicht flexibel auf geänderte Bedingungen reagiert werden. So würden bei einer solchen Vorrichtung, bei welcher nicht vorgesehen ist, sie regelmäßig zu kalibrieren, wenn man beispielsweise eine hygienische Schutzfolie zwischen das zu scannende Objekt und die Vorrichtung gibt, die erfassten Geometrien ungenauer werden. Dies steht den angestrebten Zielen bei der Weiterentwicklung von Intraoral-Scannern daher entgegen.

Der Erfindung liegt daher die Aufgabe zu Grunde, die oben beschrieben Nachteile zu überwinden.

Gelöst wird diese Aufgabe erfindungsgemäß durch ein Verfahren zum Kalibrieren der eingangs genannten Art, das gekennzeichnet ist durch das Erzeugen eines zweiten Kalibrierdatensets durch das Messen der optischen Eigenschaften der optischen Elemente, nachdem wenigstens eine transparente Schicht einer ersten Art wenigstens teilweise in einem Strahlengang der optischen Elemente angeordnet oder ausgetauscht wurde.

Gelöst wird diese Aufgabe erfindungsgemäß weiters durch ein Verfahren zum Betreiben der eingangs genannten Art, das gekennzeichnet ist durch das Verwenden eines zweiten Kalibrierdatensets, welches entsprechend dem ersten Kalibrierdatenset erzeugt wurde während eine transparente Schicht einer ersten Art wenigstens teilweise im Strahlengang der optischen Elemente angeordnet war, während des Erfassens der dreidimensionalen Geometrie des Objektes, um durch die optischen Elemente aufgenommene und/oder projizierte zweidimensionale Oberflächencharakteristika des Objektes rechnerisch zu entzerren, wenn wenigstens eine transparente Schicht der ersten Art wenigstens teilweise im Strahlengang der optischen Elemente angeordnet ist.

Wird die transparente Schicht der ersten Art ausgetauscht, kann sich bereits beim Erzeugen des ersten Kalibrierdatensets eine transparente Schicht, beispielsweise eine transparente Schicht der nullten Art, im Strahlengang befunden haben.

Durch das Erzeugen und Verwenden eines zweiten Kalibrierdatensets gelingt es, die Vorteile von Systemen, bei welchen reguläres Kalibrieren vorgesehen ist, und solchen, bei welchen kein reguläres Kalibrieren vorgesehen ist, miteinander zu verbinden. So wird für ein System, bei welchem kein reguläres Kalibrieren vorgesehen ist, die Möglichkeit geschaffen, auf eine Veränderung seiner optischen Eigenschaften zu reagieren, ohne dass dabei sowohl für den Energieverbrauch ungünstige als auch zeit- und/oder rechenintensive Kalibrierungen vorgenommen werden müssen, sondern es kann zwischen den zuvor erzeugten Kalibrierdatensets gewechselt werden. Die besonderen Vorteile von Systemen mit besonders geringem Energieverbrauch werden beispielsweise in der WO2013/116880 und der WO2013/116881 näher diskutiert und die vorliegende Erfindung kann mit Vorteil bei den in der WO2013/116880 und WO2013/116881 beschriebenen Systemen verwendet werden.

Ein Kalibrierdatenset enthält dabei wenigstens einen Kalibrierdatensatz. Ein Kalibrierdatensatz kann beispielsweise erzeugt werden, indem ein bekanntes Objekt vermessen wird. Dieses kann beispielsweise spezielle Muster aufweisen oder eine Ebene sein, die aus verschiedenen Winkeln gemessen wird. In einer Weiterbildung kann, gegebenenfalls unter Verwendung des dabei entstandenen Kalibrierdatensatzes, das Objekt erneut aus einer anderen Entfernung aber vorzugsweise unter ansonsten gleichen Bedingungen vermessen werden, wodurch ein weiterer Kalibrierdatensatz erzeugt wird. Alle erzeugten Kalibrierdatensätze werden dann zu einem Kalibrierdatenset zusammengefügt. Werden hinreichend viele Kalibrierdatensätze aus unterschiedlichen Abständen erzeugt, kann das Kalibrierdatenset so beispielsweise auch die Tiefenkompensation umfassen.

Eine bevorzugte Durchführungsform des Verfahrens zum Kalibrieren beinhaltet weiters das Erzeugen wenigstens eines weiteren Kalibrierdatensets durch das Messen dieser optischen Eigenschaften der optischen Elemente, nachdem wenigstens eine transparente Schichteiner weiteren Art wenigstens teilweise in einem Strahlengang der optischen Elemente angeordnet oder ausgetauscht wurde.

So können für eine beliebige Anzahl verschiedener Arten von Schichten korrespondierende Kalibrierdatensets beispielweise bereits durch den Hersteller der Vorrichtung erzeugt werden. Ein entsprechendes System kann dadurch beim Betreiben beispielsweise durch einen Zahnarzt oder Zahntechniker optimale Flexibilität bieten.

Dementsprechend beinhaltet eine bevorzugte Durchführungsform des Verfahrens zum Betreiben das Verwenden eines weiteren Kalibrierdatensets, welches entsprechend dem ersten Kalibrierdatenset erzeugt wurde, während eine transparente Schicht einer weiteren Art wenigstens teilweise im Strahlengang der optischen Elemente angeordnet war, während des Erfassens der dreidimensionalen Geometrie des Objektes, um durch die optischen Elemente aufgenommene und/oder projizierte zweidimensionale Oberflächencharakteristika des Objektes rechnerisch zu entzerren, wenn wenigstens eine transparente Schicht der weiteren Art wenigstens teilweise im Strahlengang der optischen Elemente angeordnet ist.

In einer weiteren bevorzugten Durchführungsform des Verfahrens zum Betreiben wird erkannt, ob eine transparente Schicht wenigstens teilweise im Strahlengang angeordnet ist und gegebenenfalls welcher Art diese Schicht ist und abhängig davon wird automatisch ein korrespondierendes Kalibrierdatenset verwendet. So wird bevorzugt immer jeweils das richtige Kalibrierdatenset verwendet.

In einer bevorzugten Weiterbildung der Erfindung erfolgt das Erkennen, beispielsweise über einen Sensor, automatisch. Hierfür kann beispielweise an einer Schutzhülle, welche über einen Scannerkopf der Vorrichtung zum optischen Erfassen der dreidimensionalen Geometrie von Objekten gegeben wird und die dabei die transparente Schicht im Strahlengang bildet, eine Markierung bzw. Kennzeichnung vorgesehen sein, die durch einen Sensor an der Vorrichtung erkannt wird.

In einer weiteren bevorzugten Weiterbildung der Erfindung kann vorgesehen sein, dass, wenn keine Schicht erkannt wird, zu der ein korrespondierendes Kalibrierdatenset hinterlegt ist, kein Erfassen der dreidimensionalen Oberflächengeometrie erfolgt. So kann unterstützt werden, dass keine Aufnahmen ohne eine Schutzhülle gemacht werden, wie es beispielsweise durch Hygienevorschriften vorgegeben sein kann.

In einer besonders bevorzugten Weiterbildung des erfindungsgemäßen Verfahrens zum Kalibrieren wird vor dem Erzeugen eines Kalibrierdatensets die transparente Schicht auf eine Betriebstemperatur der Vorrichtung gebracht. Dies ist vor allem dann besonders vorteilhaft, wenn es sich bei der Vorrichtung um eine Vorrichtung handelt, die über eine Beleuchtungseinrichtung verfügt, oder sich aus einem anderen Grund auf eine Betriebstemperatur erwärmt, die über der Umgebungstemperatur liegt. Insbesondere intra-oral Scanner, die über eine Beleuchtungseinrichtung verfügen, können sich im Betrieb stark erwärmen. Eine über einen solchen intra-oral Scanner gegebene Schutzhülle erwärmt sich im Betrieb zusammen mit dem Scanner. Da diese Schutzhülle üblicherweise aus einem Polymer, Silikat oder dergleichen besteht, ist damit zu rechnen, dass sich bei einem Wechsel der Temperatur der Schutzhülle auch die optischen Eigenschaften der Schutzhülle verändern, insbesondere die optische Dichte und die Dicke der Hülle.

Weitere bevorzugte Durchführungsformen der Erfindung sind Gegenstand der übrigen Unteransprüche.

Nachstehend ist ein bevorzugtes Ausführungsbeispiel der Erfindung anhand der Zeichnungen näher beschrieben. Es zeigt:
- Fig. 1: einen schematisierten Umriss eines Scannergehäuses, welches partiell von einer Schutzhülle umgeben ist,
- Fig. 2: einen schematisierten inneren Aufbau eines Scanners,
- Fig. 3: einen schematisierten Strahlenverlauf durch eine Optik des Scanners,
- Fig. 4: den schematisierten Ablauf beim Erzeugen von Kalibrierdatensätzen,
- Fig. 5: einen vereinfachten Ablauf für das Erkennen,
- Fig. 6: einen vereinfacht dargestellten, erweiterten Ablauf für das Erkennen und
- Fig. 7: einen optionalen zusätzlichen Vorgang für den Ablauf von -Fig. 6.

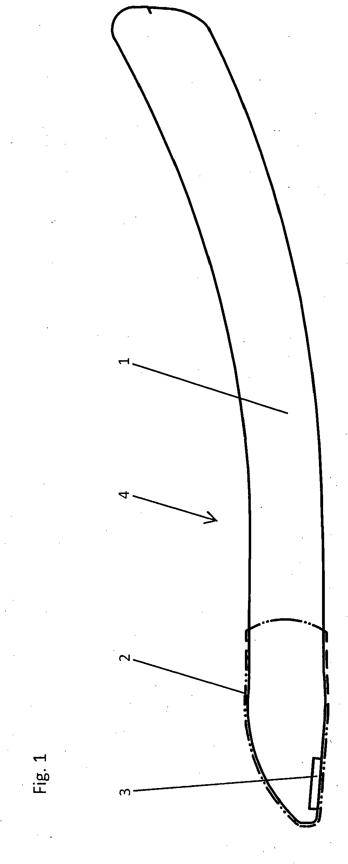

Fig. 1 zeigt einen schematisierten Umriss eines Scannergehäuses 1 eines Scanners 4, welches partiell von einer Schutzhülle 2 umgeben ist, die strichpunktiert dargestellt ist. Der Scanner 4 ist in dem dargestellten Beispiel eine Vorrichtung zum optischen Erfassen der dreidimensionalen Geometrie von Objekten wie Zähnen oder anderen intra-oralen Strukturen. Die Schutzhülle 2 bildet eine transparente Schicht im Strahlengang von optischen Elementen des Scanners 4 bzw. der Vorrichtung. Weiters ist innerhalb des Scannergehäuses 1 schematisch eine transparente Abdeckung 3 dargestellt, welchein der dargestellten Ausführungsform des Scanners 4 in das .Gehäuse integriert ist. Die Abdeckung 3 kann beispielsweise aus Glas oder transparentem Kunststoff bestehen oder dieses bzw. diesen beinhalten. Durch die Abdeckung 3 kann der Scanner 4 abgeschlossen gestaltet werden. Dies hat zum einen den Vorteil, dass der Scanner einfach gereinigt werden kann. Zum anderen wird so ein Beschlagen der optischen und elektronischen/elektrischen Bestandteile des Scanners, beispielsweise durch kondensierte Atemluft eines Patienten, verhindert. Damit können qualitativ bessere Aufnahmen gemacht werden und gegebenenfalls empfindliche Bauteile werden gegen eine Beeinträchtigung bzw. Beschädigung durch Feuchtigkeit geschützt.

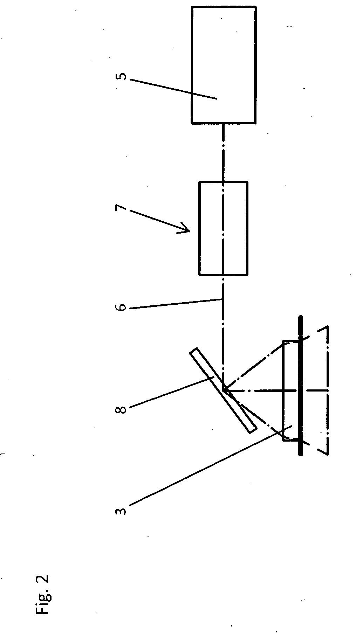

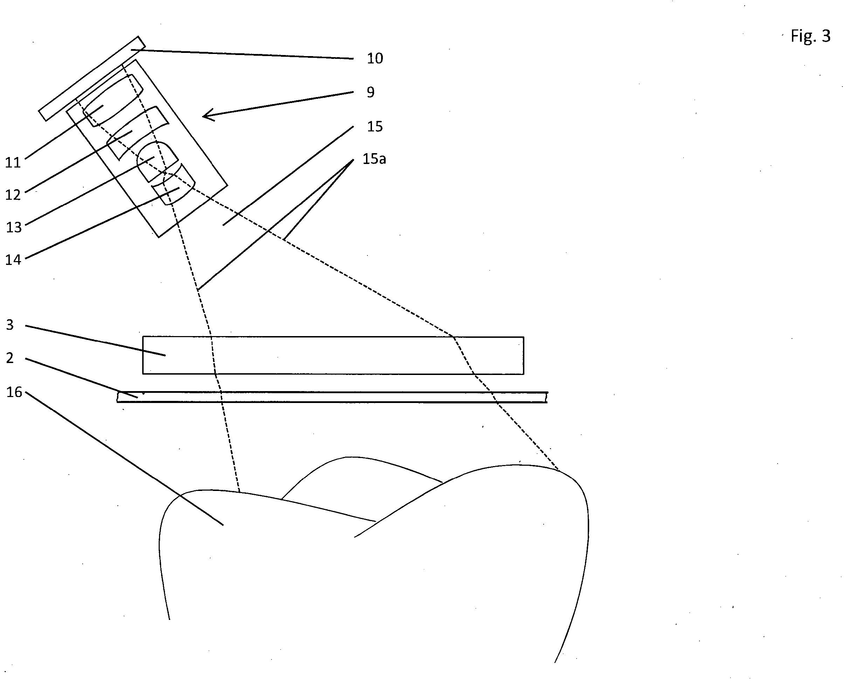

Fig. 2 zeigt einen beispielhaften, grob schematisierten inneren Aufbau des Scanners 4. Eine Lichtquelle 5 erzeugt Licht, dessen Verlauf durch die Linie 6 grob symbolisiert wird. Eine das Licht bündelnde und ausrichtende Optik 7 ist ebenfalls nur symbolisch dargestellt und kann verschiedene optische Elemente 11, 12, 13, 14 (siehe Fig. 3) enthalten. Weiters befindet sich in der dargestellten Ausführungsform im Gehäuse 1 ein Umlenkspiegel 8. Dieser dient vor allem dazu, die einzelnen Bestandteile des Scanners 4 so in diesem anordnen zu können, dass der Scanner 4 ergonomisch angenehm gestaltet werden kann.

Fig. 3 zeigt einen schematisierten Strahlenverlauf durch eine Aufnahmeoptik 9 des Scanners 4. Von einem Sensor 10 aus betrachtet, durchlaufen die Lichtstrahlen die Optik 7. Der im Wesentlichen kegelförmige Strahlengang 15 ist symbolisch durch die ihn begrenzenden strichlierten Linien 15a dargestellt. In der Optik können verschiedene beispielhaft dargestellte, optische Elemente 11, 12, 13, 14 angeordnet sein. Bei der dargestellten Aufnahmeoptik 9 sind die optischen Elemente 11, 12, 13, 14 Linsen, die Aufnahmeoptik 9 kann aber auch zusätzlich oder alternativ beispielsweise Blenden, Filter, Spiegel (plan oder auch gewölbt) und/oder dergleichen beinhalten. In Blickrichtung des Sensors10 befindet sich nach der Optik 9 zunächst leerer Raum und dann die Abdeckung 3. Diese verändert den Verlauf des Strahlenganges 15 was auch durch den nicht gradlinigen Verlauf der Linien 15a angedeutet wird.

Nach der Abdeckung 3 folgt die Schutzhülle 2, welche den Strahlenverlauf ein weiteres Mal verändert. Abhängig von den optischen Eigenschaften der Schutzhülle 2 kann diese Änderung unterschiedlich ausfallen. Gemäß der Erfindung können daher nur ein Kalibrierdatenset für eine einzige (erste) Art von Schutzhüllen 2 aber auch unterschiedliche weitere Kalibrierdatensets für Schutzhüllen mit unterschiedlichen optischen Eigenschaften erzeugt werden. Der dargestellte Abstand zwischen der Abdeckung 3 und der Schutzhülle 2 dient lediglich der Veranschaulichung und wird in der realen Anwendung der Erfindung nicht oder kaum vorhanden sein, da er nur schwer zu kontrollieren ist und ein direktes Anliegen der Schutzhülle 2 an der Abdeckung 3 daher wünschenswert ist. Ein definierter Abstand, beispielsweise mittels Abstandhaltern, ist aber natürlich auch realisierbar.

Nach der Schutzhülle 2 erfasst der Sichtkegel ein abzubildendes Objekt 16. Das Objekt 16 ist im dargestellten Beispiel ein Zahn 16. Für den Strahlengang einer bevorzugt vorhandenen Beleuchtung gelten analog ähnliche Bedingungen.

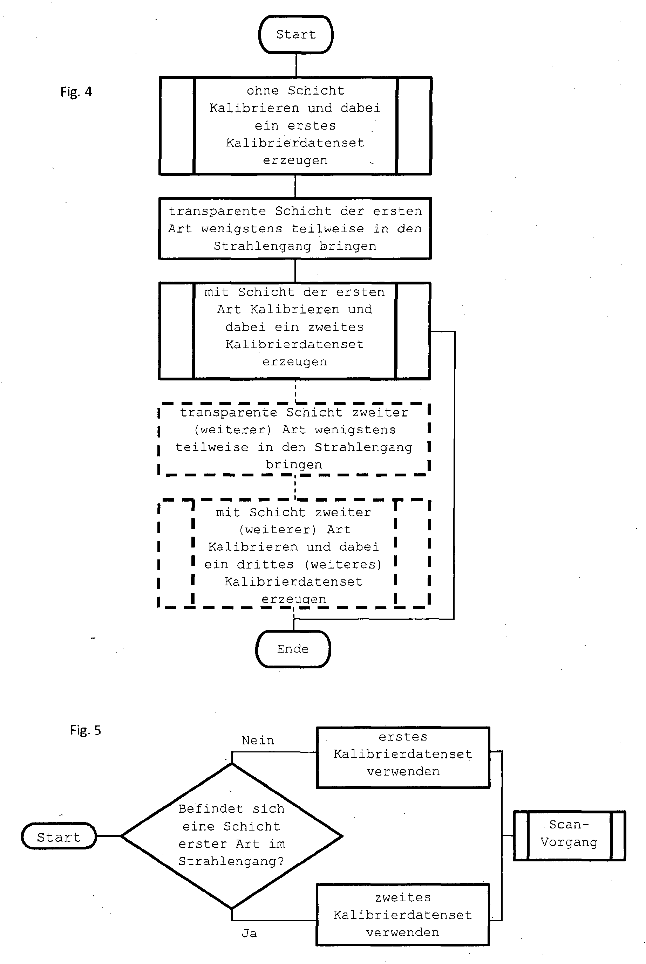

Fig. 4 zeigt beispielhaft den schematisierten Ablauf beim Erzeugen von Kalibrierdatensets für eine Durchführungsform des Verfahrens, bei welcher vorgesehen ist, dass ein Kalibrierdatenset für einen Betrieb ohne eine transparente Schicht erzeugt wird. Dabei wird der Scanner zunächst ohne Schutzhülle kalibriert, wobei ein erstes Kalibrierdatenset erzeugt wird. Dabei werden die Eigenschaften der optischen Elemente des Scanners erfasst. Bevorzugt werden dabei alle Eigenschaften des Scanners, sowohl für den projizierenden als auch den sensorischen Anteil des Scanners, erfasst. So kann nach Bereinigen/Korrigieren der durch den Sensor bzw. die Sensoren erfassten Daten mittels des Kalibrierdatensets vorzugsweise von idealen Bedingungen ausgegangen werden, das heißt einer punktförmigen Lichtquelle und eineridealen Lochkamera. Selbstverständlich können die erfindungsgemäßen Verfahren auch angewendet werden, um rechnerisch andere Bedingungen zu schaffen, beispielsweise Scheimpflugbedingungen.

Nachdem eine transparente Schicht der ersten Art in den Strahlengang gebracht wurde, wird der Kalibriervorgang wiederholt und dabei ein zweites Kalibrierdatenset erzeugt. Die erzeugten Kalibrierdaten werden dann auf einem geeigneten Speichermedium der Vorrichtung oder eines Computersystems zum Steuern der Vorrichtung hinterlegt. Alternativ können die Kalibrierdaten auch auf einem von der Vorrichtung oder dem Computersystem unabhängigen Speichermedium, welches durch die Vorrichtung und/oder das Computersystem auslesbar ist, hinterlegt werden. So können auch bei geänderten optischen Eigenschaften die durch den Sensor erfassten Daten derart korrigiert/bereinigt werden, dass für die Berechnung der Oberflächengeometrie des Objektes von idealen Bedingungen ausgegangen werden kann.

Optional kann der Kalibriervorgang für beliebig viele weitere Arten von transparenten Schichten wiederholt werden. Für jede Art wir dabei ein eigenes Kalibrierdatenset erzeugt und hinterlegt. Bei Ausführungsformen, bei denen die Kalibrierdatensets auf externen Speichermedien hinterlegt werden, kann beispielsweise, wenn die Art der transparenten Schicht geändert wird, auch das Speichermedium geändert werden. In einer Weiterbildung könnte auch jede Schutzhülle mit einem Chip versehen sein, auf welchem die für die Art der Schutzhülle spezifischen Änderungen der optischen Eigenschaften hinterlegt sind und der durch den Scanner ausgelesen werden kann.

Alternativ kann bevorzugt auch für jedes Kalibrierdatenset ein eigenes Speichermedium vorgesehen sein. In einer Weiterbildung dieser Ausführungsform können dann die Speichermedien, beispielsweise USB-Sticks, Markierungen aufweisen, die mit Markierungen an den Schutzhüllen übereinstimmen. Schutzhüllen mit einem roten Punkt können beispielsweise mit dem roten Speichermedium korrespondieren, Schutzhüllen mit blauem Punkt mit einem blauen Speichermedium usw.

In einer weiteren Weiterbildung der Erfindung können Kalibrierdatensets auch vollständig oder teilweise über eine Fernverbindung, beispielsweise eine Internetverbindung, übermittelt werden. So kann eine Vorrichtung beim Nutzer, beispielsweise über eine Softwareaktualisierung, weitere Kalibrierdaten erhalten. Dies ist insbesondere dann besonders sinnvoll, wenn beispielsweise eine neue Schutzhüllenart entwickelt wird bzw. auf den Markt kommt. So kann die Vorrichtung mit neuen Schutzhüllenarten betrieben werden, auch wenn bei der Auslieferung der Vorrichtung noch keine Kalibrierdaten für diese Schutzhüllenarten erstellt werden konnten, ohne dass diese Kalibrierdaten vor Ort erfasst werden müssen. Für diese Durchführungsform ist es besonders vorteilhaft, wenn die Kalibrierdatensets so aufgebaut sind, dass zwischen vorrichtungsspezifischen Kalibrierdaten und solchen, welche die transparente Schicht betreffen, unterschieden werden kann.

In einer weiteren optionalen Durchführungsform des erfindungsgemäßen Verfahrens kann auch vollständig auf ein Kalibrieren ohne transparente Schichten verzichtet werden, so dass es nur möglich ist, kalibrierte und damit hinreichend zuverlässige Aufnahmen zu machen, wenn eine Schutzhülle verwendet wird. Wenn beispielsweise Hygienevorschriften eine Verwendung des Scanners ohne Schutzhülle untersagen, können so diese Vorschriften unterstützt werden. In einer zusätzlichen gegebenenfalls auch unabhängig von der gegenständlichen Erfindung bevorzugten Weiterbildung kann seitens des Systems eine Nutzung des Scanners auch grundsätzlich unterbunden werden, wenn durch das System erkannt wird, dass sich keine Schutzhülle über dem Scannerkopf befindet.

Fig. 5 zeigt einen vereinfachten Ablauf für das Erkennen, welches Kalibrierdatenset verwendet werden soll. Dabei erfolgt zunächst eine Abfrage, ob sich eine transparente Schicht im Strahlengang befindet. Dies kann automatisch, beispielsweise, über einen dafür vorgesehenen Sensor, oder auch durch Eingabe einer Bedienperson erfolgen. Je nach Ergebnis der Abfrage wird dann das erste Kalibrierdatenset verwendet, wenn sich keine transparente Schicht im Strahlengang befindet, und das zweite Kalibrierdatenset, wenn sich eine transparente Schicht im Strahlengang befindet. Der Scannvorgang kann dann unter Verwendung des ausgewählten Kalibrierdatensets wie aus dem Stand der Technik an sich bekannt erfolgen.

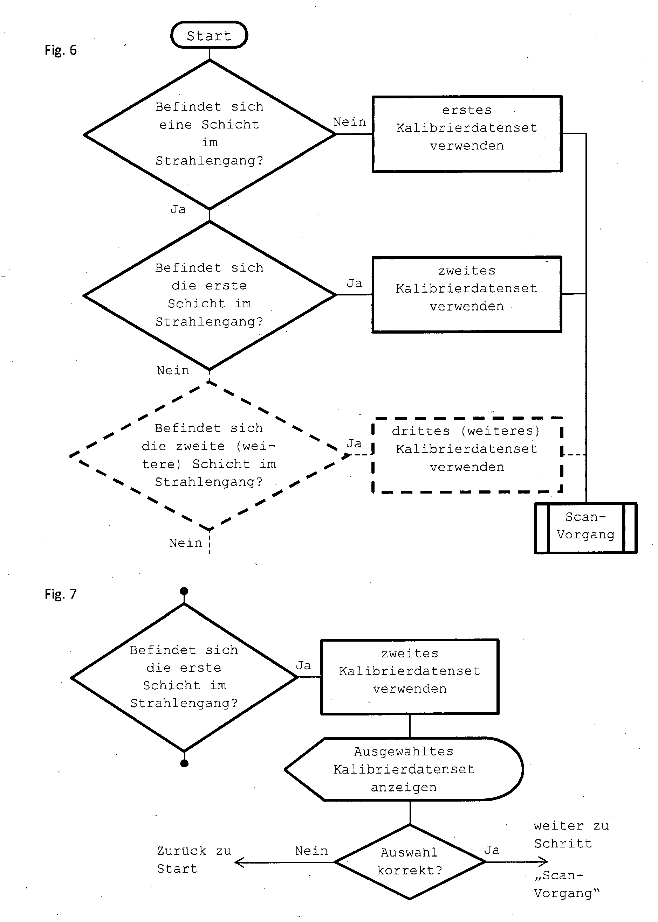

Fig. 6 zeigt einen vereinfachten erweiterten Ablauf für das Erkennen, welches Kalibrierdatenset verwendet werden soll. Dabei wird zunächst wie in Fig. 5 abgefragt, ob sich eine transparente Schicht im Strahlengang befindet. Dies kann automatisch erfolgen. Wurde keine Schicht erkannt, wird das erste Kalibrierdatenset verwendet und der Scan-Vorgang kann erfolgen. Wird eine Schicht erkannt wird abgefragt ob es sich dabei um eine Schicht der ersten Art handelt. Wenn eine Schicht der ersten Art erkannt wurde, wird der Scan-Vorgang mit dem zweiten Kalibrierdatenset durchgeführt. Diese Abfrage kann je nach Anzahl von hinterlegten Kalibrierdatensets und verschiedenen Arten von transparenten Schichten beliebig erweitert werden.

In einer Weiterbildung der Erfindung kann das Erkennen vollständig automatisiert erfolgen. Hierfür kann beispielsweise an der Schutzhülle eine Markierung vorgesehen sein, die vom Scanner erkannt wird. Dafür kann die Schutzhülle beispielsweise einen RFID-Chip oder einen Strichcode enthalten, welcher durch ein entsprechendes Element im oder am Scanner erkannt wird.

Fig. 7 zeigt einen optionalen zusätzlichen Vorgang für den Ablauf von Fig. 6. Dabei wird, nachdem eine Schicht erkannt und das entsprechende Kalibrierdatenset ausgewählt wurde, das ausgewählte Kalibrierdatenset angezeigt. Darauf folgt die Abfrage, ob das richtige Kalibrierdatenset ausgewählt wurde. Ist dem Nutzer bekannt, welches Kalibrierdatenset zu verwenden ist, beispielsweise anhand einer Typenbezeichnung auf der Schutzhülle, kann er diese Auswahl bestätigen, worauf der Scan-Vorgang folgt. Wurde eine falsche Schutzhülle erkannt oder beispielsweise auch die Anwesenheit einer Schutzhülle erkannt, obwohl keine Schutzhülle über den Scannerkopf gegeben wurde, wird die Abfrage verneint und es folgen erneut die Schritte ab "Start" wie in Fig. 6 dargestellt. Analog kann dieser Vorgang auch auf andere entsprechende Schritte in Fig. 6 oder Fig. 5 angewendet werden.

Alternativ kann die Auswahl natürlich auch manuell, über eine Schnittstelle, beispielsweise eine graphische Benutzeroberfläche (GUI) auf einem Bildschirm, erfolgen.

## Patentansprüche

1. Verfahren zum Kalibrieren einer Vorrichtung (4) zum optischen Erfassen der dreidimensionalen Geometrie von Objekten (16), wobei optische Eigenschaften von optischen Elementen (11, 12, 13, 14) der Vorrichtung (4) vor dem Erfassen gemessen und auf einem Speichermedium hinterlegt werden, wodurch ein erstes Kalibrierdatenset erzeugt wird, und wobei das erste Kalibrierdatenset während des Erfassens der dreidimensionalen Geometrie des Objektes (16) verwendet wird, um durch die optischen Elemente (11, 12, 13, 14) aufgenommene und/oder projizierte, zweidimensionale Oberflächencharakteristika des Objektes (16) rechnerisch zu entzerren, **gekennzeichnet durch** das Erzeugen eines zweiten Kalibrierdatensets durch das Messen der optischen Eigenschaften der optischen Elemente (11, 12, 13, 14), nachdem wenigstens eine transparente Schicht (2) einer ersten Art wenigstens teilweise in einem Strahlengang (15) der optischen Elemente (11, 12, 13, 14) angeordnet oder ausgetauscht wurde.

2. Verfahren nach Anspruch 1, **gekennzeichnet durch** das Erzeugen wenigstens eines weiteren Kalibrierdatensets durch das Messen dieser optischen Eigenschaften der optischen Elemente (11, 12, 13, 14), nachdem wenigstens eine transparente Schicht (2) einer weiteren Art wenigstens teilweise in einem Strahlengang (15) der optischen Elemente (11, 12, 13, 14) angeordnet oder ausgetauscht wurde.

3. Verfahren nach Anspruch 1 oder 2, **dadurch gekennzeichnet, dass** vor dem Erzeugen eines Kalibrierdatensets die transparente Schicht (2) auf eine Betriebstemperatur der Vorrichtung gebracht wird.

4. Verfahren zum Betreiben einer Vorrichtung (4) zum optischen Erfassen der dreidimensionalen Geometrie von Objekten (16), wobei optische Eigenschaften von optischen Elementen (11, 12, 13, 14) der Vorrichtung (4) vor dem Erfassen gemessen und auf einem Speichermedium hinterlegt wurden, wodurch ein erstes Kalibrierdatenset erzeugt wurde, und wobei das erste Kalibrierdatenset während des Erfassens der dreidimensionalen Geometrie des Objektes (16) verwendet wird, um durch die optischen Elemente (11, 12, 13, 14) aufgenommene und/oder projizierte, zweidimensionale Oberflächencharakteristika des Objektes (16) rechnerisch zu entzerren, **gekennzeichnet durch** das Verwenden eines zweiten Kalibrierdatensets, welches entsprechend dem ersten Kalibrierdatenset erzeugt wurde während eine transparente Schicht (2) einer ersten Art wenigstens teilweise im Strahlengang (15) der optischen Elemente (11, 12, 13, 14) angeordnet war, während des Erfassens der dreidimensionalen Geometrie des Objektes (16), um durch die optischen Elemente (11, 12, 13, 14) aufgenommene und/oder projizierte zweidimensionale Oberflächencharakteristika des Objektes (16) rechnerisch zu entzerren, wenn wenigstens eine transparente Schicht (2) der ersten Art wenigstens teilweise im Strahlengang (15) der optischen Elemente (11, 12, 13, 14) angeordnet ist.

5. Verfahren nach Anspruch 4, **gekennzeichnet durch** das Verwenden eines weiteren Kalibrierdatensets, welches entsprechend dem ersten Kalibrierdatenset erzeugt wurde, während eine transparente Schicht (2) einer weiteren Art wenigstens teilweise im Strahlengang (15) der optischen Elemente (11, 12, 13, 14) angeordnet war, während des Erfassens der dreidimensionalen Geometrie des Objektes (16), um durch die optischen Elemente (11, 12, 13, 14) aufgenommene und/oder projizierte, zweidimensionale Oberflächencharakteristika des Objektes (16) rechnerisch zu entzerren, wenn wenigstens eine transparente Schicht (2) der weiteren Art wenigstens teilweise im Strahlengang (15) der optischen Elemente (11, 12, 13, 14) angeordnet ist.

6. Verfahren nach Anspruch 4 oder 5, **dadurch gekennzeichnet, dass** erkannt wird, ob eine transparente Schicht (2) wenigstens teilweise im Strahlengang (15) angeordnet ist und gegebenenfalls welcher Art diese Schicht (2) ist, und dadurch, dass abhängig davon automatisch ein korrespondierendes Kalibrierdatenset verwendet wird.

7. Verfahren nach Anspruch 6, **dadurch gekennzeichnet, dass** das Erkennen, beispielsweise über einen Sensor, automatisch erfolgt.

8. Verfahren nach Anspruch 6 oder 7, **dadurch gekennzeichnet, dass**, wenn keine transparente Schicht (2) erkannt wird, zu der ein korrespondierendes Kalibrierdatenset hinterlegt ist, kein Erfassen der dreidimensionalen Oberflächengeometrie erfolgt.

9. Vorrichtung (4) zum optischen Erfassen der dreidimensionalen Geometrie von Objekten (16), insbesondere intra-oral Scanner, mit einem Speichermedium, wobei das Speichermedium wenigstens zwei Kalibrierdatensets enthält, **dadurch gekennzeichnet, dass** die Vorrichtung (4) ein Mittel zum Erkennen des Vorhandenseins einer transparenten Schicht (2) im Strahlengang aufweist und eingerichtet ist, das entsprechende Kalibrierdatenset auszuwählen.

10. Vorrichtung (4) zum optischen Erfassen der dreidimensionalen Geometrie von Objekten (16), insbesondere intra-oral Scanner, mit einem Speichermedium, wobei das Speichermedium wenigstens zwei Kalibrierdatensets enthält, **dadurch gekennzeichnet, dass** die Vorrichtung (4) ein Mittel zum Identifizieren einer transparenten Schicht (2) im Strahlengang aufweist und eingerichtet ist, das entsprechende Kalibrierdatenset auszuwählen.

11. Vorrichtung (4) zum optischen Erfassen der dreidimensionalen Geometrie von Objekten (16), insbesondere intra-oral Scanner, mit einem Speichermedium, wobei das Speichermedium wenigstens zwei Kalibrierdatensets enthält, **dadurch gekennzeichnet, dass** die Kalibrierdatensets sich auf das Vorhandensein einer transparent Schicht im Strahlengang beziehen und dass die Auswahl eines Kalibrierdatensets manuell über eine Schnittstelle, beispielsweise eine graphische Benutzeroberfläche, GUI, auf einem Bildschirm, erfolgt.

12. Vorrichtung nach einem der Ansprüche 9 bis 11, **dadurch gekennzeichnet, dass** die Kalibrierdatensets nach Anspruch 1 oder 2 gewonnen wurden.

13. Computersystem zum Steuern einer Vorrichtung (4) zum optischen Erfassen der dreidimensionalen Geometrie von Objekten (16), insbesondere eines intra-oral Scanners, **dadurch gekennzeichnet, dass** das Computersystem programmiert ist, die Verfahrensschritte nach einem der Ansprüche 1 bis 7 auszuführen.

14. Verwendung der Vorrichtung (4) nach Anspruch 9 bis 12 zum Ausführen des Verfahrens nach einem der Ansprüche 4 bis 8.

## Claims

1. A method for calibrating a device (4) for optically detecting the three-dimensional geometry of objects (16), wherein optical properties of optical elements (11, 12, 13, 14) of the device (4) are measured prior to the detection and stored on a memory medium, thereby generating a first calibration data set, and wherein the first calibration data set is used during the detection of the three-dimensional geometry of the object (16) to computationally equalize two-dimensional surface characteristics of the object (16) that are recorded and/or projected by the optical elements (11, 12, 13, 14), **characterized by** the generation of a second calibration data set by measuring the optical properties of the optical elements (11, 12, 13, 14) after at least one transparent layer (2) of a first type has been at least partially situated and/or replaced in a beam path (15) of the optical elements (11, 12, 13, 14) .

2. The method according to Claim 1, **characterized by** the generation of at least one further calibration data set by measuring these optical properties of the optical elements (11, 12, 13, 14) after at least one transparent layer (2) of a further type has been at least partially situated and/or replaced in a beam path (15) of the optical elements (11, 12, 13, 14).

3. The method according to Claim 1 or 2, **characterized in that** the transparent layer (2) is brought to an operating temperature of the device prior to generating a calibration data set.

4. A method for operating a device (4) for optically detecting the three-dimensional geometry of objects (16), wherein optical properties of optical elements (11, 12, 13, 14) of the device (4) are measured prior to the detection and stored on a memory medium, thereby having generated a first calibration data set, and wherein the first calibration data set is used during the detection of the three-dimensional geometry of the object (16) to computationally equalize two-dimensional surface characteristics of the object (16) that are recorded and/or projected by the optical elements (11, 12, 13, 14), **characterized by** the use of a second calibration data set that has been generated corresponding to the first calibration data set while a transparent layer (2) of a first type was at least partially situated in the beam path (15) of the optical elements (11, 12, 13, 14), during the detection of the three-dimensional geometry of the object (16), in order to computationally equalize two-dimensional surface characteristics of the object (16) that are recorded and/or projected by the optical elements (11, 12, 13, 14), when at least one transparent layer (2) of the first type is at least partially situated in the beam path (15) of the optical elements (11, 12, 13, 14).

5. The method according to Claim 4, **characterized by** the use of a further calibration data set that has been generated corresponding to the first calibration data while a transparent layer (2) of a further type was at least partially situated in the beam path (15) of the optical elements (11, 12, 13, 14), during the detection of the three-dimensional geometry of the object (16), in order to computationally equalize two-dimensional surface characteristics of the object (16) that are recorded and/or projected by the optical elements (11, 12, 13, 14), when at least one transparent layer (2) of the further type is at least partially situated in the beam path (15) of the optical elements (11, 12, 13, 14).

6. The method according to Claim 4 or 5, **characterized in that** it is recognized whether a transparent layer (2) is at least partially situated in the beam path (15), and if applicable the type of layer (2) is recognized, and on this basis a corresponding calibration data set is automatically used.

7. The method according to Claim 6, **characterized in that** the recognition takes place automatically, for example via a sensor.

8. The method according to Claim 6 or 7, **characterized in that** if no transparent layer (2) is recognized for which a corresponding calibration data set is stored, no detection of the three-dimensional surface geometry takes place.

9. A device (4), in particular an intraoral scanner, for optically detecting the three-dimensional geometry of objects (16), having a memory medium, wherein the memory medium contains at least two calibration data sets, **characterized in that** the device (4) has a means for recognizing the presence of a transparent layer (2) in the beam path, and is configured for selecting the appropriate calibration data set.

10. A device (4), in particular an intraoral scanner, for optically detecting the three-dimensional geometry of objects (16), having a memory medium, wherein the memory medium contains at least two calibration data sets, **characterized in that** the device (4) has a means for identifying a transparent layer (2) in the beam path, and is configured for selecting the appropriate calibration data set.

11. A device (4), in particular an intraoral scanner, for optically detecting the three-dimensional geometry of objects (16), having a memory medium, wherein the memory medium contains at least two calibration data sets, **characterized in that** the calibration data sets pertain to the presence of a transparent layer in the beam path, and the selection of a calibration data set takes place manually via an interface, for example a graphical user interface (GUI) on a screen.

12. The device according to one of Claims 9 to 11, **characterized in that** the calibration data sets are obtained according to Claim 1 or 2.

13. A computer system for controlling a device (4), in particular an intraoral scanner, for optically detecting the three-dimensional geometry of objects (16), **characterized in that** the computer system is programmed to carry out the method steps according to one of Claims 1 to 7.

14. Use of the device (4) according to Claims 9 to 12 for carrying out the method according to one of Claims 4 to 8.

## Revendications

1. Procédé permettant d'étalonner un dispositif (4) pour l'acquisition optique de la géométrie tridimensionnelle d'objets (16), dans lequel des propriétés optiques d'éléments optiques (11, 12, 13, 14) du dispositif (4) sont mesurées avant l'acquisition et stockées sur un support de stockage de façon à générer un premier ensemble de données d'étalonnage, et dans lequel le premier ensemble de données d'étalonnage est utilisé lors de l'acquisition de la géométrie tridimensionnelle de l'objet (16) pour égaliser de manière arithmétique des caractéristiques de surface bidimensionnelles de l'objet (16), enregistrées et/ou projetées par les éléments optiques (11, 12, 13, 14), **caractérisé par** la génération d'un deuxième ensemble de données d'étalonnage par la mesure des propriétés optiques des éléments optiques (11, 12, 13, 14) après qu'au moins une couche transparente (2) d'un premier type a été disposée ou remplacée au moins en partie sur une trajectoire des rayons (15) des éléments optiques (11, 12, 13, 14).

2. Procédé selon la revendication 1, **caractérisé par** la génération d'au moins un ensemble de données d'étalonnage supplémentaire par la mesure de ces propriétés optiques des éléments optiques (11, 12, 13, 14) après qu'au moins une couche transparente (2) d'un type supplémentaire a été disposée ou remplacée au moins en partie sur une trajectoire des rayons (15) des éléments optiques (11, 12, 13, 14).

3. Procédé selon la revendication 1 ou 2, **caractérisé en ce qu'**avant la génération d'un ensemble de données d'étalonnage, la couche transparente (2) est portée à une température de fonctionnement du dispositif.

4. Procédé permettant d'exploiter un dispositif (4) pour l'acquisition optique de la géométrie tridimensionnelle d'objets (16), dans lequel des propriétés optiques d'éléments optiques (11, 12, 13, 14) du dispositif (4) sont mesurées avant l'acquisition et stockées sur un support de stockage de façon à générer un premier ensemble de données d'étalonnage, et dans lequel le premier ensemble de données d'étalonnage est utilisé lors de l'acquisition de la géométrie tridimensionnelle de l'objet (16) pour égaliser de manière arithmétique des caractéristiques de surface bidimensionnelles de l'objet (16), enregistrées et/ou projetées par les éléments optiques (11, 12, 13, 14), **caractérisé par** l'utilisation d'un deuxième ensemble de données d'étalonnage généré selon le premier ensemble de données d'étalonnage pendant qu'une couche transparente (2) d'un premier type était disposée au moins en partie sur une trajectoire des rayons (15) des éléments optiques (11, 12, 13, 14) pendant l'acquisition de la géométrie tridimensionnelle de l'objet (16) pour égaliser de manière arithmétique des caractéristiques de surface bidimensionnelles de l'objet (16), enregistrées et/ou projetées par les éléments optiques (11, 12, 13, 14) quand au moins une couche transparente (2) du premier type est disposée au moins en partie sur la trajectoire des rayons (15) des éléments optiques (11, 12, 13, 14).

5. Procédé selon la revendication 4, **caractérisé par** l'utilisation d'un ensemble de données d'étalonnage supplémentaire généré selon le premier ensemble de données d'étalonnage pendant qu'une couche transparente (2) d'un type supplémentaire était disposée au moins en partie sur la trajectoire des rayons (15) des éléments optiques (11, 12, 13, 14) pendant l'acquisition de la géométrie tridimensionnelle de l'objet (16) pour égaliser de manière arithmétique des caractéristiques de surface bidimensionnelles de l'objet (16), enregistrées et/ou projetées par les éléments optiques (11, 12, 13, 14) quand au moins une couche transparente (2) du type supplémentaire est disposée au moins en partie sur la trajectoire des rayons (15) des éléments optiques (11, 12, 13, 14).

6. Procédé selon la revendication 4 ou 5, **caractérisé en ce qu'**il est détecté si une couche transparente (2) est disposée au moins en partie sur la trajectoire des rayons (15), et le cas échéant, de quel type est cette couche (2), et **en ce qu'**en fonction de cela, un ensemble de données d'étalonnage correspondant est utilisé automatiquement.

7. Procédé selon la revendication 6, **caractérisé en ce que** la détection est effectuée automatiquement, par exemple à l'aide d'un capteur.

8. Procédé selon la revendication 6 ou 7 **caractérisé en ce que**, si aucune couche transparente (2) n'est détectée pour laquelle un ensemble de données d'étalonnage correspondant a été stocké, la détection de la géométrie de surface tridimensionnelle n'est pas effectuée.

9. Dispositif (4) de détection optique de la géométrie tridimensionnelle d'objets (16), en particulier un scanner intraoral, avec un support de stockage, le support de stockage contenant au moins deux ensembles de données d'étalonnage, **caractérisé en ce que** le dispositif (4) présente un moyen de détection de la présence d'une couche transparente (2) sur la trajectoire des rayons et est aménagé pour sélectionner l'ensemble de données d'étalonnage correspondant.

10. Dispositif (4) de détection optique de la géométrie tridimensionnelle d'objets (16), en particulier un scanner intraoral, avec un support de stockage, le support de stockage contenant au moins deux ensembles de données d'étalonnage, **caractérisé en ce que** le dispositif (4) présente un moyen d'identification d'une couche transparente (2) sur la trajectoire des rayons et est aménagé pour sélectionner l'ensemble de données d'étalonnage correspondant.

11. Dispositif (4) de détection optique de la géométrie tridimensionnelle d'objets (16), en particulier un scanner intraoral, avec un support de stockage, le support de stockage contenant au moins deux ensembles de données d'étalonnage, **caractérisé en ce que** les ensembles de données d'étalonnage se réfèrent à la présence d'une couche transparente sur la trajectoire des rayons, et **en ce que** la sélection d'un ensemble de données d'étalonnage est effectuée manuellement par l'intermédiaire d'une interface, en particulier d'une interface utilisateur graphique sur un écran.

12. Dispositif selon l'une quelconque des revendications 9 à 11, **caractérisé en ce que** les ensembles de données d'étalonnage ont été obtenus selon la revendication 1 ou 2.

13. Système informatique permettant de commander un dispositif (4) de détection optique de la géométrie tridimensionnelle d'objets (16), en particulier un scanner intraoral, **caractérisé en ce que** le système informatique est programmé pour effectuer les étapes de procédé selon l'une quelconque des revendications 1 à 7.

14. Utilisation du dispositif (4) selon la revendication 9 à 12 pour effectuer le procédé selon l'une quelconque des revendications 4 à 8.
